# EUROPEAN PATENT APPLICATION

(11) **EP 4 559 480 A1**
(43) Date of publication of application: **28.05.2025**
(21) Application number: 23849301.9
(22) Date of filing: 28.07.2023
(51) Int. Cl.: A61K 45/00, A61K 31/42, A61K 31/4985, A61K 31/202, A61K 31/519, A61K 31/498, A61K 38/08, A61K 38/16, A61P 9/04

(54) **USE OF JUN INHIBITOR IN PREPARING MEDICAMENT FOR TREATING HEART FAILURE WITH PRESERVED EJECTION FRACTION**

(30) Priority: 30.07.2022 CN 202210911679
(71) Applicant: Fuwai Hospital, Chinese Academy Of Medical Sciences And Peking Union Medical College, Beijing 100037 (CN)
(72) Inventor: WANG, Li, Beijing 100037 (CN); LI, Zheng, Beijing 100037 (CN)
(74) Representative: E. Blum & Co. AG
(86) International application number: PCT/CN2023/109846
(87) International publication number: WO 2024/027593

(57) **Abstract**

The present invention relates to use of a *Jun* inhibitor in preparing a medicament for treating heart failure with preserved ejection fraction (HFpEF).

The figure accompanying the Abstract is Fig. 3.

## Description

### Technical Field

The present invention relates to use of *Jun* inhibitor in preparing medicament for treating heart failure with preserved ejection fraction (HFpEF).

### Background Art

Cardiovascular disease represents one of the difficult problems in medical challenges. Heart failure (HF) is the main cause of death in patients with cardiovascular disease and a major clinical problem. Heart failure includes heart failure with reduced ejection fraction (HFrEF) and heart failure with preserved ejection fraction (HFpEF).

Currently, HFpEF accounts for about 50% of all heart failure patients, and its prevalence is increasing at an alarming rate, which is also the main cause of the increase in cardiovascular disease mortality. HFpEF is a syndrome involving multiple organ disorders, and a disorder or result jointly caused by heart, lung, kidney, bone, immunity, inflammation, metabolism and other components, which is often accompanied by symptoms such as obesity, hypertension, myocardial hypertrophy, diabetes or atrial fibrillation. HFpEF is a syndrome with high incidence rate and high mortality. According to clinical statistics, the mortality due to HF is 35%, among which the proportion of deaths caused by HFpEF accounts for 57%. However, few drug therapies or medical devices have so far been proven to change the disease progression and prognosis of HFpEF patients. At present, there is an urgent need in the field to develop a drug and/or treatment method that can effectively treat HFpEF.

*Jun* is a transcription factor and a member of the AP1 family, which has chromatin binding activity and transcriptional cis-regulatory region binding activity, and participates in the regulation of processes including animal organ development, protein phosphorylation and cell proliferation. *Jun* inhibitors refer to inhibitors that can inhibit the expression of *Jun* genes, reduce the binding activity of *Jun* to DNA, reduce the level of *Jun* gene expression products, or prevent or block *Jun* signal transduction. At present, studies have shown that *Jun* inhibitors have therapeutic effects in animal models of endometriosis, breast cancer, and sepsis, etc.

### Contents of the present invention

The present inventors of the present invention have, for the first time, discovered that inhibiting the high expression of *Jun* using *Jun* inhibitors can have a preventive and therapeutic effect on HFpEF, thereby completing the present invention.

Therefore, the technical solution of the present invention comprises: use of a *Jun* inhibitor in the manufacture of a medicament for treating or preventing heart failure with preserved ejection fraction (HFpEF).

In a preferred embodiment, the *Jun* inhibitor is selected from T-5224, MLN44, SR11302, Veratramine, KCR motif peptide-1-{N-[2-succinamidylethyl]amino}anthraquinone, NY2267, cFos LZ, anti-Jun, anti-Fos SZ, FosW, FosWCANDI, CPW, FosUisCan, A-Fos, JNK Inhibitor VIII, IQ3, Tanzisertib (CC-930), or pharmaceutically acceptable salts of the above substances. The above substances have been reported to have *Jun* inhibitor activity, and their structures are shown below:
T-5224: 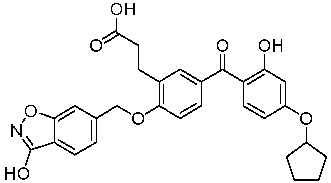
MLN944: 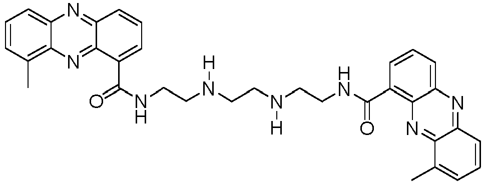
SR11302: 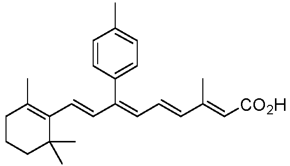
Veratramine: 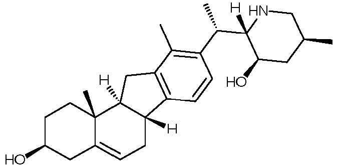
KCR motif peptide-1-{N-[2-succinamidylethyl]amino}anthraquinone: 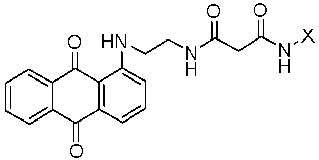
   NHX=ARCKA; AKCRA; AKSRA; AKCRNA;
   AKCRKA; AKCRNRA; AKCRKRA; AAKCRAA
   (note: in the above sequences representing NHX, the letters are single-letter abbreviations of amino acids)
NY2267: 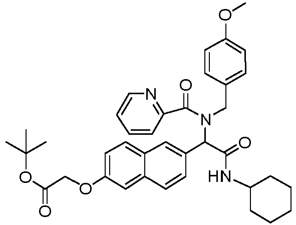
cFos LZ: 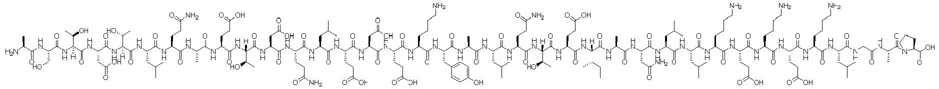
anti-Jun SZ: 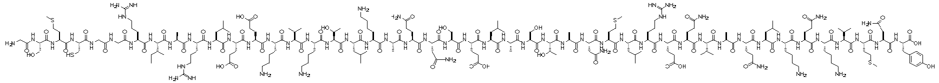
anti-Fos SZ: 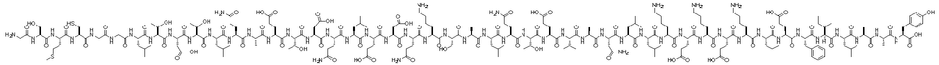
FosW: 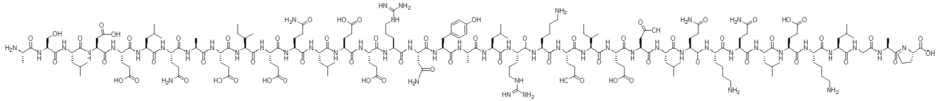
FosWCANDI: 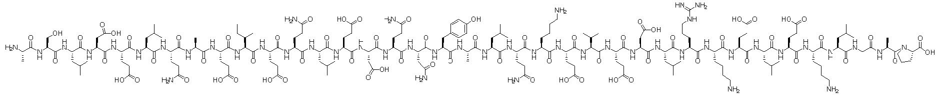
CPW: 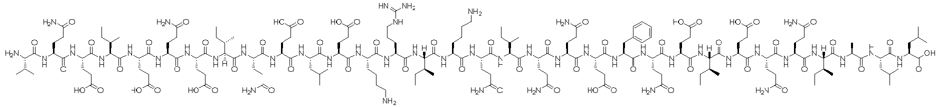
FosUisCan: 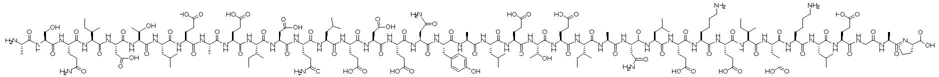
A-Fos: 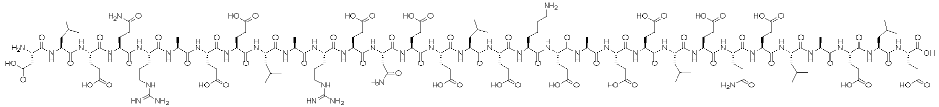
JNK Inhibitor VIII: 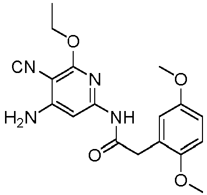
IQ3: 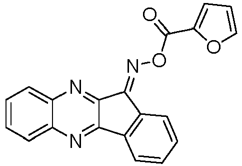
Tanzisertib (CC-930):

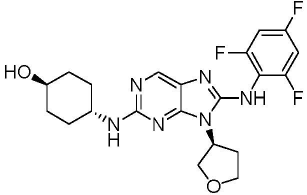

In a more preferred embodiment, the *Jun* inhibitor is T-5224 or a pharmaceutically acceptable salt thereof.

### Brief Description of the Drawings

Figure 1 shows the construction of HFpEF model. A: Schematic diagram of the experimental process; B: Detection results of systolic function of mice after 5 weeks of feeding; C: Detection results of diastolic function of mice after 5 weeks of feeding.
Figure 2 shows the relative expression of *Jun* in cardiomyocytes after 15 weeks of HFD+L-NAME feeding, indicating that *Jun* is highly expressed in HFpEF model mice.
Figure 3 shows that T-5224 could effectively alleviate the occurrence and development of HFpEF. A: Detection results of systolic function of mice at different time points; B: Detection results of diastolic function of mice at different time points; C: Changes in body weight of mice with different treatments; D: Changes in expression of *Jun* in cardiomyocytes of mice with different treatments.

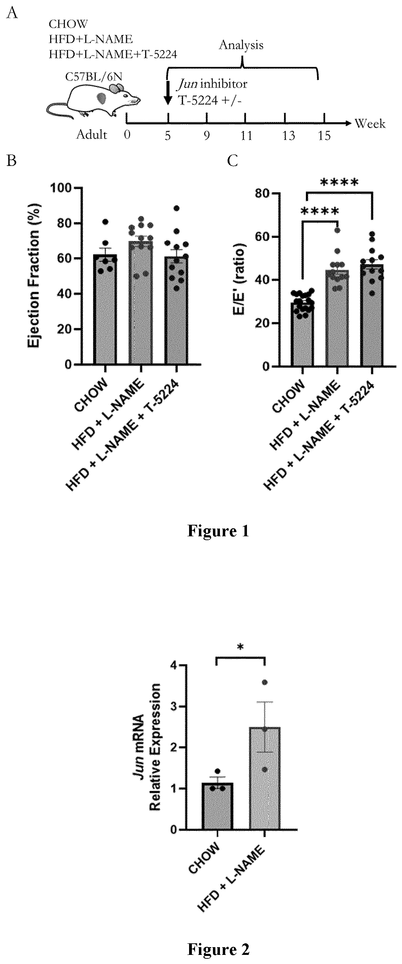

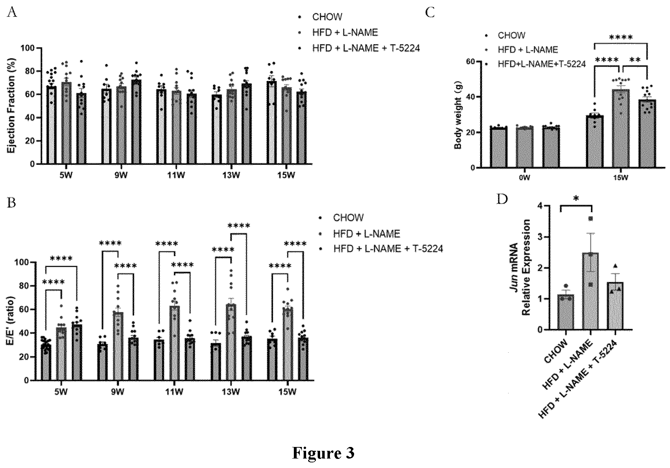

In figures 1 to 3, "CHOW" represents normal mice, "HFD+L-NAME" represents mice in model control group, and "HFD+L-NAME+T-5524" represents mice in model treatment group. In figure 3A to 3C, "CHOW" represents normal mice, "HFD+L-NAME" represents mice in model control group, and the bar charts at each time point are arranged from left to right in the order of "CHOW", "HFD+L-NAME" and "HFD+L-NAME+T-5524".

### Specific Models for Carrying Out the present invention

The following examples illustrate the specific embodiments of the present invention and verify the effects achieved by the present invention. It should be understood that the following examples are only illustrative, and the technical solution of the present invention is not limited to these examples.

### Example

### 1. Materials and reagents

In this example, C57BL/6N wild-type mice were purchased from Beijing Vital River. The sources of reagents were shown in the following table.

| Reagent | Cat. No. | Supplier |
|---|---|---|
| Collagenase type II | 17101015 | Gibico |
| T-5224 | S8966 | Selleck |
| Polyvinylpyrrolidone (PVP) | S3596 | Selleck |
| GeneJet RNA purification kit | K0732 | Thermo Scientific |
| iScript TM cDNA synthesis kit | 1708890 | Bio-Rad |
| iTaqUniversl SYBR Green supermix | 1725121 | Bio-Rad |

In addition to the above, other materials and reagents used in this example were also commercially available products.

### 2. Animal experiment guideline

In this example, all animal studies were conducted under the guidance of the Laboratory Animal Center of the Institutional Animal Care and Use Committee of Fuwai Hospital, National Center for Cardiovascular Diseases, China. All mice were propagated and raised in the same environment, and the mice were randomly divided into groups during the experiment. Echocardiographic analysis was performed by an independent researcher who was unaware of the study objectives.

### 3. Induction of model of heart failure with preserved ejection fraction

Male C57BL/6N wild-type mice aged 8 to 10 weeks were divided into three groups, namely, normal group (normal diet and drinking water), model control group (high-fat diet combined with administration of N-nitro-L-arginine methyl ester) and model treatment group (high-fat diet combined with administration of N-nitro-L-arginine methyl ester and treatment with T-5224). Among them, the model control group and the model treatment group were modeled by the following literature: Gabriele G. Schiattarella et al., Nitrosative stress drives heart failure with preserved ejection fraction, https://doi.org/10.1038/s41586-019-1100-z. Specifically, heart failure with preserved ejection fraction was induced by a high-fat diet (HFD) (60% kcal, from fat (lard)) and N-nitro-L-arginine methyl ester (abbreviated as L-NAME, 0.5 g/L in drinking water) to obtain the HFpEF animal model.

In the fifth week of model induction, the systolic function parameter LVEF of mice did not change, while the diastolic function parameter (E/E') increased significantly in the 5^{th} week of model induction, indicating that the model of heart failure with preserved ejection fraction recorded in the above literature had been successfully obtained. At the same time, there was no significant difference in the diastolic function parameter (E/E') between the model control group and the model treatment group in the 5^{th} week. Subsequent drug administration was carried out under the same baseline, as shown in Figure 1.

### 4. Jun expression was correlated with HFpEF

In the 15^{th} week of model induction, the cardiomyocytes of normal group and the model control group were isolated by perfusion method, and quantitative RCR detection was performed. The specific operations were as follows:
4.1. Isolation of adult mouse cardiomyocytes:
   In order to isolate cardiomyocytes from the heart of adult mice, we used the classic perfusion method to isolate cardiomyocytes. Specifically, mice were injected with 100 µl of sodium heparin (1000 units in 50 ml) 20 minutes before being killed to prevent heart coagulation during the operation, which increased the difficulty of digestion. After that, the mice were anesthetized and killed, and the hearts were removed and transferred to a calcium-free solution for washing. Then, the Langendorff method was used for digestion. The heart was perfused with a calcium-free solution for 5 minutes using a Langendorff apparatus, and then digested with digestive enzyme solution (0.7 mg/ml type II collagenase and 0.7 mg/ml bovine serum albumin in a calcium-free solution) for about 30 minutes. The heart was constantly touched at about 20 minutes. When the heart became soft and slippery, which indicated that the digestion was basically completed, the tissues from the ventricles were then collected, cut into pieces, and gently pipetted to dissociate into single cells. The cells were allowed to settle, and the supernatant was taken to remove the undigested and adherent tissues, and centrifuged at 100 g and 4°C for 2 minutes to obtain cardiomyocyte pellets, and the supernatant was mostly non-cardiomyocytes. Cardiomyocytes were resuspended in a calcium-free solution containing 10% FBS for subsequent experiments. Non-cardiomyocytes could be resuspended with culture medium or PBS for subsequent experiments. If purer cardiomyocytes and non-cardiomyocytes were required, the cell suspension could be centrifuged (100g, 2 minutes at room temperature) three times to separate cardiomyocytes from non-cardiomyocytes. Cardiomyocytes were collected for further experiments.
4.2. Quantitative PCR detection:
   Total RNA was extracted from cells using GeneJet RNA purification kit (Thermo Scientific, K0732), and 0.1µg of total RNA was reverse transcribed using iScript TM cDNA synthesis kit (Bio-Rad, 1708890) to generate cDNA. qPCR was performed using iTaqUniversl SYBR Green supermix (1725121, Bio-Rad) on ABI Vii7 real-time system (Life Technologies, Q6), and b-Actin was used for standardized quantitative analysis. As shown in Figure 2, compared with normal mice, high expression of *Jun* was significantly observed in the mouse HFpEF animal model. This indicated that the expression of *Jun* in mice was correlated with HFpEF, and *Jun* was highly expressed in HFpEF.

### 5. T-5224 administration method

After the animal model was obtained, that was, from the 5^{th} week of inducing the HFpEF model, the model treatment group was treated with T-5224, the model control group was treated with a solvent without drug, and the mice with normal diet and drinking water during the whole induction process were used as negative control. The mice were administrated with drugs when they were raised for 5 weeks. For the treatment group, T-5224 was administered at 250 mg/kg according to the body weight of mice. Specifically, the administration was performed every other day, 0.8 mg of T-5224 was dissolved in 200 uL of 1% PVP solution each time. The administration started from the 5^{th} week and ended at the 13^{th} week (a total of 15 administrations), with a total dose of 250 mg/kg. The control group was administrated with an equal volume of 1% PVP solution, and other treatments were the same.

### 6. Conventional echocardiography and Doppler imaging

All the mice were raised under different conditions for five weeks and then began to undergo conventional echocardiography and Doppler imaging, which was performed every two weeks until the end of the 15^{th} week. Specifically, transthoracic echocardiography was performed using the VisualSonics Vevo 2100 system equipped with an MS400 transducer (Visual Sonics). Left ventricular ejection fraction (LVEF) and other systolic function indices were obtained from the short-axis M-mode scans at the midventricular level, as indicated by the presence of papillary muscles, in conscious, gently restrained mice. Cardiac apical four-chamber views were obtained in the anesthetized mice, and used for diastolic function measurements by pulsed waves and tissue Doppler imaging at the level of mitral valve. Anesthesia was induced by 2.5% isoflurane and confirmed by lack of response to firm pressure on one of the hind paws. During echocardiographic acquisition (under temperature-controlled conditions), isoflurane was reduced to 1.0% to 1.5% and adjusted to maintain heart rate in the range of 500 beats per minute. The parameters collected included: heart rate, left ventricular end-diastolic diameter, left ventricular end-systolic diameter, end-diastolic interventricular septal wall thickness, left ventricular end-diastolic posterior wall, left ventricular fractional shortening, LVEF, peak Doppler blood inflow velocity across mitral valve during early diastole, peak Doppler blood inflow velocity across mitral valve during late diastole, isovolumetric relaxation time, peak tissue Doppler of myocardial relaxation velocity at the mitral valve annulus during early diastole and early filling deceleration time. At the end of the procedure, all the mice recovered from anesthesia without any abnormalities. All parameters were measured at least 3 times, and the mean values were provided. The echocardiography and Doppler imaging included systolic function and diastolic function detection.

### 7. Experimental results and conclusions

Firstly, the detection of systolic function and diastolic function of mice in the 5^{th} week showed that there was no significant change in systolic function, but the diastolic function parameter E/E' increased significantly, proving that there was a disorder in diastolic function, indicating that the model described in the above literature was successfully obtained. At the same time, taking the 5^{th} week as the starting point of drug administration, there was no significant difference in cardiac diastolic function between the model control group and the model treatment group before drug administration (Figure 1A-C), and the drug administration was carried out on this basis.

Secondly, compared with the normal group, the expression of *Jun* in the model control group was upregulated, indicating that the expression of *Jun* was correlated with HFpEF. In the HFpEF mouse model, *Jun* was highly expressed (Figure 2). Based on this correlation, it could be judged that *Jun* inhibitor could be used for the prevention and treatment of HFpEF.

Further, T-5224 was used to verify the effectiveness of *Jun* inhibitor in preventing and treating HFpEF. After confirming that the model construction was successful and that the baselines of the model control group and the model treatment group were consistent, the model treatment group was treated with T-5224. The cardiac function detection results showed that in the model treatment group (after T-5224 administration), the occurrence and development of HFpEF had been well suppressed. Specifically, after treatment with T-5224, the diastolic function in the mice treated with high-fat diet combined with L-NAME (HFD + 0.5g/L L-NAME) was significantly improved and could be maintained until the 15^{th} week. However, in the model control mice that were not treated with T-5224, continued deterioration of diastolic function was observed (Figure 3A-B). At the same time, in the model treatment group, the expression of *Jun* was down-regulated compared with the model control group (Figure 3D), and the mouse obesity was improved (Figure 3C). This indicated that T-5224, as a *Jun* inhibitor, could bring about preventive and therapeutic effects on HFpEF in the mouse HFpEF model.

## Claims

1. Use of a *Jun* inhibitor in the manufacture of a medicament for treating or preventing heart failure with preserved ejection fraction.

2. The use according to claim 1, wherein the *Jun* inhibitor is selected from T-5224, MLN44, SR11302, Veratramine, KCR motif peptide-1-{N-[2-succinamidylethyl]amino}anthraquinone, NY2267, cFos LZ, anti-Jun, anti-Fos SZ, FosW, FosWCANDI, CPW, FosUisCan, A-Fos, JNK Inhibitor VIII, IQ3, Tanzisertib (CC-930), or pharmaceutically acceptable salts of the above substances.

3. The use according to claim 2, wherein the *Jun* inhibitor is selected from T-5224 or a pharmaceutically acceptable salt thereof.
